# EUROPÄISCHE PATENTSCHRIFT

(11) **EP 1 562 882 B1**
(45) Veröffentlichungstag und Bekanntmachung des Hinweises auf die Patenterteilung: **20.01.2010**
(21) Anmeldenummer: 03789012.6
(22) Anmeldetag: 06.11.2003
(51) Int. Cl.: C07C 29/17, B01J 23/656, B01J 23/42

(54) **VERBESSERTER KATALYSATOR UND VERFAHREN ZUR HERSTELLUNG VON ALKOHOLEN DURCH HYDRIERUNG AN DIESEM KATALYSATOR**
IMPROVED CATALYST AND METHOD FOR PRODUCING ALCOHOL BY HYDROGENATION ON SAID CATALYST
CATALYSEUR AMELIORE ET PROCEDE DE PRODUCTION D'ALCOOLS PAR HYDROGENATION SUR CE CATALYSEUR

(30) Priorität: 11.11.2002 DE 10252280
(43) Veröffentlichungstag der Anmeldung: 17.08.2005
(73) Patentinhaber: BASF SE, 67056 Ludwigshafen (DE)
(72) Erfinder: SCHUBERT, Markus, 67063 Ludwigshafen (DE); HUBER-DIRR, Sylvia, 64673 Zwingenberg (DE); HESSE, Michael, 67549 Worms (DE); PFEIFER, Jochen, 76831 Ilbesheim (DE); FISCHER, Rolf-Hartmuth, 69121 Heidelberg (DE); RÖSCH, Markus, 55276 Dienheim (DE); BOTTKE, Nils, 68165 Mannheim (DE); WECK, Alexander, 67251 Freinsheim (DE); WINDECKER, Gunther, 67067 Ludwigshafen (DE); HEYDRICH, Gunnar, 67117 Limburgerhof (DE)
(86) Internationale Anmeldenummer: PCT/EP2003/012378
(87) Internationale Veröffentlichungsnummer: WO 2004/043890

(56) Entgegenhaltungen:
- EP-A- 0 848 991
- DE-A- 2 519 817
- DE-A- 10 009 817
- US-A- 4 149 962
- BAZIN D ET AL: "In situ study by XAS of the sulfidation of industrial catalysts: The Pt and PTRe/Al2O3 systems" APPLIED CATALYSIS A: GENERAL, ELSEVIER SCIENCE, AMSTERDAM, NL, Bd. 162, Nr. 1-2, 18. November 1997 (1997-11-18), Seiten 171-180, XP004338233 ISSN: 0926-860X

## Beschreibung

Die Erfindung betrifft ein Verfahren zur Herstellung von Alkoholen durch Hydrierung von Carbonylgruppen enthaltende Verbindungen an rheniumhaltigen Trägerkatalysatoren sowie die verbesserten rheniumhaltigen Trägerkatalysatoren.

Die industrielle Hydrierung von Carbonygruppen enthaltenden Edukten wie Aldehyden, Ketonen, Carbonsäuren, Carbonsäureanhydriden und Estern an rheniumhaltigen Katalysatoren zu Alkoholen ist an sich bekannt.

DE-A 2 519 817 beschreibt Katalysatoren für die die Hydrierung von Maleinsäureanhydrid, Maleinsäure oder deren Mischungen, die gleichzeitig Elemente der VII und VIII Nebengruppe des Periodensystems der Elemente enthalten. Bevorzugt weisen die Katalysatoren Rhenium und Palladium auf, die im Verlauf der Katalysatorherstellung gemäß den Ausführungsbeispielen bevorzugt, gleichzeitig auf den Träger aufgebracht werden. Es ist gemäß der Offenbarung der DE-A 2 519 817 auch möglich die Palladiumverbindung zuerst auf den Träger aufzubringen.

Nach einem Trocknungsschritt entstehen so geträgerte Palladium-Rhenium-Katalysatoren, deren Aktivität in Hydrierungen von Carbonylgruppen enthaltenden Verbindung so gering ist, dass die gleichzeitige Anwendung von hohen Drucken und hohen Temperaturen von 215 bis 230°C erforderlich wird. Die Durchführung der Hydrierungen bei hohen Drucken und hohen Temperaturen ist bedingt durch hohe Energie- und Materialkosten wenig wirtschaftlich. Zudem nimmt die Korrosivität insbesondere bei Einsatz von Carbonsäure-Lösungen unter diesen Bedingungen_zu.

Palladium-Rhenium-Katalysatoren weisen bei einigen Hydrierreaktionen, wie beispielsweise der Hydrierung von Carbonsäuren oder deren Derivaten zu Alkoholen, weiterhin den Nachteil auf, dass durch das Palladium Nebenreaktionen wie die Etherbildung begünstigt werden.

Die Vermeidung von Ethern als Nebenprodukt technischer Hydrierprozesse hat sich DE-A 100 09 817 zum Ziel gesetzt. Die durch dass gleichzeitige Aufbringen von Rhenium- und Palladiumverbindungen auf einen Aktivkohleträger und anschließende Trocknungsschritte hergestellten Katalysatoren erweisen sich als wesentlich selektiver. Die erhaltenen Katalysatoren sind jedoch so inaktiv, dass sehr hohe Edelmetallbeladungen des Trägers von mehr als 8 Gew.-% Platin und mehr als 14,5 Gew.-% Rhenium erforderlich sind, um die gewünschte Aktivität zu erhalten, was das in DE-A 100 09 817 offenbarte Verfahren insgesamt unwirtschaftlich erscheinen lässt.

Eine Aufgabe der vorliegenden Erfindung war es, Verfahren zur katalytischen Hydrierung von Carbonylverbindungen zu Alkoholen sowie Katalysatoren bereitzustellen, die die Nachteile des Standes der Technik nicht aufzuweisen. Weiterhin sollen die Katalysatoren geeignet sein, mit hoher Ausbeute und Selektivität Carbonylverbindungen in der Flüssigphase zu Alkoholen zu hydrieren.

Demgemäss betrifft die vorliegende Erfindung einen Katalysator enthaltend 0,1 bis 20 Gew.-% Rhenium und 0,05 bis 10 Gew.-% Platin, bezogen auf die Gesamtmasse des Katalysators aus Trägermaterial und katalytischer Aktivmasse, auf einem Träger, erhältlich durch ein Verfahren, bei dem man
a) den gegebenenfalls vorbehandelten Träger mit einer Lösung einer Rheniumverbindung behandelt,
b) trocknet und in einer reduktiven Atmosphäre bei 80 bis 600°C tempert,
c) mit einer Lösung einer Platinverbindung imprägniert und erneut trocknet.

Als Träger bzw. Trägermaterial werden in den erfindungsgemäßen Katalysator im allgemeinen Metalloxide wie die Oxide des Aluminiums und Titans, Zirkondioxid, Hafniumdioxid, Siliciumdioxid, gegebenenfalls vorbehandelte Aktivkohle auch oder gegebenenfalls vorbehandelte graphitische Kohleträger, Nitride, Silicid, Carbid oder Borid, verwendet. Bei der erwähnten Vorbehandlung kann es sich um eine oxidative Vorbehandlung, wie sie beispielsweise im EP-A 848 991 beschrieben ist, handeln. Eine nicht oxidative Vorbehandlung des Trägermaterials, beispielsweise mit Phosphorsäure, ist aus DE-A 100 09 817 bekannt. Bevorzugt werden Träger aus Titandioxid, Zirkondioxid, Hafniumdioxid, gegebenenfalls vorbehandelter Aktivkohle und/oder graphitischem Kohlenstoff verwendet.

Als Rheniumkomponente wird üblicherweise (NH₄)ReO₄, Re₂O₇, ReO₂, ReCl₃, ReCl₅, Re(CO)₅Cl, Re(CO)₅Br oder Re₂(CO)₁₀ verwendet, ohne dass diese Aufzählung ausschließlich gemeint ist. Bevorzugt wird Re₂O₇ eingesetzt.

Es wird neben Rhenium vorzugsweise noch Platin auf den Katalysator aufgebracht. Das Platin kann als z.B. Platinpulver, Oxid, Oxidhydrat, Nitrat, Platin(II)- oder -(IV)-chlorid, Platin(IV)-chlorwasserstoffsäure, Platin(II)- oder -(IV)-Bromid, Platin(II)-jodid, cis- bzw. trans-Platin(II)-diamin-chlorid, cis- bzw. trans-Platin(IV)-diamin-chlorid, Platin(II)diamin-nitrit, Platin(II)-ethylendiamin-chlorid, Platin(II)-tetraminchlorid bzw. - chlorid-Hydrat, Platin(II)-tetraminnitrat, Platin(II)-ethylendiamin-chlorid, Platin(0)-tetrakis-(triphenylphosphin), cis- bzw. trans-Platin(II)-bis-(triethylphosphin)-chlorid, cis- bzw. trans-Platin(II)-bis-(triethylphosphin)oxalat, cis-Platin(II)-bis-(triphenylphosphin)-chlorid, Platin(IV)-bis-(triphenylphosphin)-oxid, Platin(II)(-2,2'-6',2"-terpyridin)chlorid-Dihydrat, cis-Platin-bis-(acetonitril)-dichlorid, cis-Platin-bis-(benzonitril)-dichlorid, Platin(II)-acetylacetonat, Platin(11)-1c,5c-cyclooctadien-chlorid bzw. -bromid, Platinnitrosylnitrat, bevorzugt als Platinoxid oder -nitrat, besonders bevorzugt als Platinnitrat aufgebracht werden, ohne daß diese Aufzählung ausschließlich gemeint ist.

Rhenium (als Metall gerechnet) ist in einer Menge von 0,1 bis 20 Gew.-%, bevorzugt 0,5 bis 13 Gew.-%, besonders bevorzugt 2 und 7 Gew.-%, bezogen auf die Gesamtmasse des Katalysators aus katalytischer Aktivmasse und Trägermaterial, vorhanden. Der erfindungsgemäße Katalysator weist Platin (als Metall gerechnet) in einer Menge von 0,05 bis 10 Gew.-%, bevorzugt 0,1 bis 8 Gew.-%, besonders bevorzugt 0,3 bis 4 Gew.-%, bezogen auf die Gesamtmasse des Katalysators aus katalytischer Aktivmasse und Trägermaterial, auf.

Das Gewichtsverhältnis von Rhenium zu Platin (als Metalle gerechnet) liegt in einem Bereich von 0,01 - 100. Bevorzugt 0,05 bis 50, besonders bevorzugt 0,1 bis 10.

Es können auf dem Katalysator noch weitere Elemente vorhanden sein. Beispielhaft seien Sn, Zn, Cu, Ag, Au, Ni, Fe, Ru, Mn, Cr, Mo, W und V genannt. Diese Elemente modifizieren den Katalysator im wesentlichen bzgl. Aktivität und Selektivität (Hydrogenolyseprodukte) sind aber nicht essentiell. Ihr Gewichtsverhältnis zu Re kann 0 bis 100, bevorzugt 0,5 bis 30, besonders bevorzugt 01, bis 5 betragen.

Die Aufbringung der Aktivkomponenten Rhenium (Re) und gegebenenfalls Platin (Pt) kann durch Imprägnierung in jeweils einem oder mehreren Schritten innerhalb der Verfahrensstufen a) oder c) mit einer wässrigen, alkoholischen oder mit anderen organischen Lösungsmitteln hergestellten Lösung der jeweiligen gelösten Verbindungen (beispielsweise Salze, Oxide, Hydroxide), Imprägnierung mit einer Lösung von gelöstem oxidischen oder metallischen Kolloid der Aktivkomponente, Gleichgewichtsadsorption in einem oder mehreren Schritten der in wässriger oder alkoholischer Lösung gelösten Salze oder Gleichgewichtsadsorption von gelöstem metallischem oder oxidischem Kolloid an der vorbehandelten Aktivkohle vorgenommen werden.

Das Imprägnieren selbst kann dabei sowohl durch Tränken mit den Lösungen als auch durch Aufsprühen erfolgen. Weiterhin kann der Träger mit Lösungen thermisch leicht zersetzbarer Salze, z.B. mit Nitraten oder thermisch leicht zersetzbaren Komplexverbindungen, z.B. Carbonyl- oder Hydrido-Komplexen der katalytisch aktiven Elemente, zu imprägnieren und den so getränkten Träger zwecks thermischer Zersetzung der adsorbierten Metallverbindungen auf Temperaturen von 300 bis 600°C zu erhitzen. Diese thermische Zersetzung kann unter einer Schutzgasatmosphäre vorgenommen. Geeignete Schutzgase sind z.B. Stickstoff, Kohlendioxid, Wasserstoff oder die Edelgase.

Die Trocknungsschritte in den Stufen a) bis c) werden im allgemeinen bei Raumtemperatur bis 120°C durchgeführt. Getempert wird vorzugsweise bei 150 bis 450°C, besonders bevorzugt bei 250 bis 375°C. Unter reduktiver Atmosphäre wird in dieser Anmeldung eine Atmosphäre verstanden, die zumindest einen Anteil an einem reduzierend wirkenden Gas wie Ammoniak, Hydrazin, C₂- bis C₆-Olefin, Kohlenmonoxid und/oder Wasserstoff aufweist, wobei Wasserstoff bevorzugt ist. Besonders bevorzugt werden als reduzierende Atmosphäre Wasserstoff/Stickstoff-Gemische eingesetzt, deren Wasserstoffanteil 10 bis 50 Gew.-% beträgt.

Es hat sich als wirtschaftlich erwiesen und ist daher bevorzugt nach Schritt b) des Verfahrens den erhaltenen Katalysatorrohling mit einem sauerstoffhaltigen Gas zu passivieren. Die Temperatur ist für den Passivierungsvorgang nicht kritisch, sollte jedoch 120°C nicht übersteigen. Im allgemeinen wird bei Raumtemperatur passiviert.

Die Katalysatoren werden üblicherweise vor ihrem Einsatz aktiviert. Diese Aktivierung kann durch Anwendung einer reduzierend wirkenden Gasatmosphäre auf den Katalysator geschehen. Bevorzugt wird eine Aktivierung mit Hilfe von Wasserstoff angewendet. Die Aktivierungstemperatur liegt dabei üblicherweise bei 100 - 500°C, bevorzugt 150 - 4500C, besonders bevorzugt 200 - 4000C. Alternative Reduktionsmethoden sind die Reduktion der metallischen Komponenten durch in Kontaktbringen mit einem flüssigen Reduktionsmittel wie Hydrazin, Formaldehyd oder Natriumformiat. Dabei werden die flüssigen Reduktionsmittel üblicherweise bei Temperaturen zwischen 10 und 100□C in Kontakt gebracht. Besonders bevorzugt ist das Inkontaktbringen bei Temperaturen zwischen 20 bis 80□C.

Als Ausgangsstoffe für die Hydrierung sind im allgemeinen Carbonylverbindungen geeignet, die zusätzlich C-C-Doppel- oder Dreifachbindungen enthalten können. Beispiel für Aldehyde sind Propionaldehyd, Butyraldehyde, Crotonaldehyd, Ethylhexanal, Nonanal und Glucose. Beispiele für Carbonsäuren sind Bernsteinsäure, Fumarsäure, Maleinsäure, Glutarsäure, Adipinsäure, 6-Hydroxycapronsäure, Octandisäure, Dodecandisäure, 2-Cyclododecylpropionsäure und gesättigte oder ungesättigte Fettsäuren. Als Ester sind Ester der vorgenannten Säuren, z.B. als Methyl-, Ethyl-, Propyl- oder Butylester zu nennen, ferner sind Lactone, z.B. gamma-Butyrolacton, delta-Valerolacton oder Caprolacton einsetzbar. Außerdem können Anhydride wie Bernsteinsäureanhydrid oder Maleinsäureanhydrid verwendet werden. Bevorzugte Ausgangsstoffe sind Bernsteinsäure, Maleinsäure, Glutarsäure, Adipinsäure, 2-Cyclododecylpropionsäure, Bernsteinsäureanhydrid, Maleinsäureanhydrid sowie die Ester dieser Säuren und gamma-Butyrolacton. Es können selbstverständlich auch Gemische von Aldehyden, Carbonsäuren, Estern, Anhydriden und/oder Lactonen, bevorzugt Gemische von Carbonsäuren, besonders bevorzugt Gemische aus Maleinsäure, Glutarsäure und/oder Adipinsäure, eingesetzt werden.

Die zu hydrierenden Verbindungen können in Substanz oder in Lösung hydriert werden. Als Lösungsmittel bieten sich bevorzugt Zwischen- und Hydrierprodukte selbst, wie zum Beispiel gamma-Butyrolacton, an, oder es werden Stoffe eingesetzt wie Alkohole wie Methanol, Ethanol, Propanol oder Butanol, ferner sind Ether wie THF oder Ethylenglycolether geeignet. Ein bevorzugtes Lösungsmittel ist Wasser, insbesondere bei der Hydrierung von Carbonsäuren.

Die Hydrierung kann in der Gas- oder Flüssigphase, ein- oder mehrstufig ausgeübt werden. In der Flüssigphase ist sowohl die Suspensions- als auch die Festbettfahrweise möglich. Die Durchführung in der Flüssigphase ist bevorzugt. Bei exothermen Reaktionen kann die Wärme durch außenliegende Kühlmittel abgeführt werden (z.B. Röhrenreaktor). Ferner ist Siedekühlung im Reaktor möglich, vor allem wenn ohne Produktrückführung hydriert werden. Bei Produktrückführung bietet sich ein Kühler im Rückführstrom an.

Erfindungsgemäß wurde erkannt, dass besonders vorteilhafte Ergebnisse bei der Herstellung von Alkoholen durch katalytische Hydrierung von Carbonylverbindungen im erfindungsgemäßen Verfahren erzielt werden, wenn der mit dem Katalysator befüllte Hydrierreaktor mit Wasser oder einer verdünnten wässrigen Lösung der Carbonylverbindung, die auch andere unter Reaktionsbedingungen inerte Lösungsmittel enthalten kann, unter Hydrierbedingungen angefahren wird. Unter Anfahren wird dabei die Inbetriebnahme des Katalysators verstanden. Dabei kann es sich bei der Inbetriebnahme um die erstmalige eines Reaktors, aber auch um ein Wiederinbetriebnehmen, beispielsweise nach Anlagenstillstand zum Zweck der Wartung mit frischem oder regenerierten Katalysator, handeln. Das Anfahren wird dabei unter den Reaktionsbedingungen der Hydrierung bezüglich Druck und Temperatur durchgeführt und erfordert im allgemeinen 30 min. bis 20 h, bevorzugt 1 bis 5 h. Die verdünnte wässrige Lösung der Carbonylverbindung enthält maximal 5 Gew.-% derselben.

Die Hydrierung wird üblicherweise bei 80 bis 210°C, bevorzugt bei 80 bis 170°C, besonders bevorzugt bei 100 bis 150°C, durchgeführt. Dabei wird üblicherweise bei einem Reaktionsdruck zwischen 20 und 230 bar, bevorzugt 50 und 220 bar, besonders bevorzugt 70 bis 160 bar, hydriert. Die Katalysatorbelastung ist abhängig von der E-delmetallkonzentration des Katalysators, liegt aber im allgemeinen höher als 0,05 g/mh.

Die im erfindungsgemäßen Verfahren erhaltenen Alkohole werden z.B. als Lösemittel und Zwischenprodukte eingesetzt. Diole wie Butandiol finden als Diolkomponente in Polyestern Verwendung.

Das erfindungsgemäße Verfahren wird anhand der nachstehenden Beispiele näher erläutert.

### Beispiele

Die angegebenen Gehalte der einzelnen Komponenten in den Hydrierausträgen sind gaschromatographisch ermittelt worden. Sie sind, wenn nicht anderes angegeben, lösungsmittelfrei gerechnet.

### Verwendete Abkürzungen

MS = Maleinsäure, MSA = Maleinsäureanhydrid, BS = Bernsteinsäure, BSA = Bernstein-säureanhydrid, GBL = gamma-Butyrolacton, BDO = 1,4-Butandiol, THF = Tetrahydrofuran, BuOH = Butanol, C-Bilanz: Geringere Werte als 100% ergeben sich vor allem durch gasförmige Nebenprodukte, die nicht durch die Analytik erfasst werden, hauptsächlich n-Butan. Bei höheren Anteilen MS/MSA oder BS/BSA wird die Analytik dieser Komponenten verfälscht, so dass hier ein zu hoher BS/BSA-Werte angezeigt wird, was zu einer C-Bilanz von deutlich über 100% führt. Die Analytik der übrigen Komponenten bleibt hiervon unberührt. In den Beispielen wurden überhöhte BS/BSA-Werte nicht korrigiert, sondern wie gemessen angegeben.

### Beispiel 1: Katalysator A

ZrO₂ Stränge (XZ 16509 der Firma Norton, Chem Process Prod. Corp., Akron (OH), USA, BET-Oberfläche 82 m²/g, Porenvolumen 0,28 cm³/g) wurden zu 1.2 - 2 mm großem Splitt gebrochen. 200 g dieses Trägermaterials wurde mit 40 ml einer wässrigen Lösung von Re₂O₇ (10 Gew.-% Re₂O₇) unter Zusatz von weiteren 140 ml Wasser 3 h behandelt. Sodann wurde das Material bei 111 °C und einem Druck von 30 - 50 mbar 2 h getrocknet. Danach wurde 30 min unter 200 Nl/h N₂ auf 150°C aufgeheizt und 60 min gehalten. Anschließend wurde für 60 min ein Gemisch aus 100 Nl/h H₂ und 100 Nl/h N₂ zugeführt, binnen 30 min auf 300°C aufgeheizt und die Bedingungen für weitere 3 h gehalten. Sodann wurde der Vorkatalysator in dieser reduzierenden Atmosphäre aus auf 50°C und anschließend in reinem N₂ (200 Nl/h) bis auf Raumtemperatur abgekühlt. Das Material wurde mit einem Gemisch von 10 Nl/h O₂ in 180 Nl/h N₂ während 14 h passiviert und schließlich mit 39,24 g einer Platin-Nitrat-Lösung (16,25 Gew.-% Pt) mit Wasser auf 160 ml getränkt. Nach 3 h Einwirkzeit wurde bei 100°C und einem Druck von 30 bis 50 mbar 2 h getrocknet. Der fertige Katalysator wurde reduziert. Der Katalysator A enthielt 2,9 Gew.% Rhenium und 2,9 Gew.-% Platin.

### Beispiele 2 und 3, Vergleichsbeispiel 1

25 ml des Katalysators A wurden in einen kontinuierlichen Rohrreaktor eingebaut. Der Feed (Eduktgemisch) bestand aus einer Maleinsäurelösung (10 Gew.-% Maleinsäure) und 50 Nl/h Wasserstoff. Die Hydrierung wurde bei 160°C und 80 bar durchgeführt. Der Flüssigaustrag wurde mittels Gaschromatographie analysiert. Die Werte in Tabelle 1 sind jeweils als Ausbeuten in [%] pro mol Maleinsäure angegeben.

**Tabelle 1:**

| Bsp. | Belastung [g_{(MS)}/ml_{(Kat)}*h] | Umsatz MS [%] | BDO [%] | GBL [%] | THF [%] | BuOH [%] | BS/BSA [%] | C-Bilanz [%] |
|---|---|---|---|---|---|---|---|---|
| V1 | 0,05 | 100 | 58,6 | 0,0 | 0,7 | 17,9 | 0,0 | 79,4 |
| 2 | 0,1 | 100 | 88,8 | 0,1 | 0,1 | 8,7 | 0,0 | 99,5 |
| 3 | 0,2 | 100 | 72,5 | 14,4 | 0,9 | 6,3 | 0,0 | 97,5 |

### Beispiel 4: Katalysator B

ZrO₂ Stränge (XZ 16509, der Firma Norton Process Prod. Corp., Akron (OH), USA, 82 m²/g, Porenvolumen 0,28 cm³/g) wurden zu 0,1 - 1 mm großem Splitt gebrochen. 200 g des Trägermaterials wurden mit 8 g Re₂O₇ in 360 ml Wasser 2 h bei 100°C behandelt und bei einem Druck von 50 mbar getrocknet. Sodann wurde das Material 60 min unter 50 Nl/h N₂ auf 300°C aufgeheizt. Ab einer Innentemperatur von 280°C wurden zusätzlich 10 Nl/h H₂ zudosiert. Nach 90 min wurde der H₂-Strom auf 25 Nl/h erhöht. Die Temperatur wurde noch 2 h gehalten, danach wurde der Vorkatalysator in dieser reduzierenden Atmosphäre auf Raumtemperatur abgekühlt. Nach Spülen mit 100 Nl/h Stickstoff für 1 h wurde das Material abschließend mit einem Gemisch von 5 Nl/h Sauerstoff in 100 Nl/h N₂ 2 h passiviert. Dann wurden 36,9 g einer Platin-Nitrat-Lösung (16,25 Gew.-% Pt) mit Wasser auf 320 ml aufgefüllt und der Vorkatalysator mit dieser Lösung getränkt. Bei 100°C und einem Druck von 50 mbar wurde das Lösungsmittel, abgetrennt und der fertige Katalysator reduziert. Der Katalysator enthielt 2.0 Gew.-% Re und 2,3 Gew.-% Pt.

### Beispiele 5 und 6

25 ml des Katalysators B wurden in einen kontinuierlichen Rohrreaktor eingebaut. Der Feed bestand aus einer Maleinsäurelösung (10 Gew.-% Maleinsäure) und wurde mit 25 g/h dosiert. Es wurde bei 160°C und 80 bar hydriert. Der Flüssigaustrag wurde mittels Gaschromatographie analysiert. Die Werte in Tabelle 2 sind jeweils als Ausbeuten in [%] pro mol Maleinsäure angegeben.

**Tabelle 2:**

| Bsp. | p [bar] | T [°C] | H₂ [Nl/h] | Umsatz MS [%] | BDO [%] | GBL [%] | THF [%] | BuOH [%] | BS/BSA [%] | C-Bilanz [%] |
|---|---|---|---|---|---|---|---|---|---|---|
| 5 | 80 | 160 | 50 | 100 | 91,4 | 0,7 | 0,1 | 4,4 | 0 | 98,3 |
| 6 | 90 | 150 | 25 | 100 | 91,1 | 1,8 | 0,3 | 6,6 | 0 | 101,9 |

### Vergleichsbeispiel 2: Katalysator V2

658 g ZrO₂ Stränge (XZ 16509 der Firma Norton Process Prod. Corp., Akron (OH), USA, 82 m²/g, Porenvolumen 0,28 cm³/g) wurden mit einem Gemisch aus 27,3 g Re₂O₇ und 131,3 g Pt-Nitratlösung (16 Gew.-% Pt) in 90 ml Wasser belegt. Das Material wird bis zur Antrocknung gut durchmischt und anschließend 16 h bei 120°C getrocknet. Danach wurde das Material in einer Reduktionskolonne bei 270°C in einem Gemisch aus 10 Nl/h Wasserstoff und 50 Nl/h Stickstoff 8 h lang reduziert, in dem Reaktionsgemisch abgekühlt und unter fließendem Stickstoff in Wasser ausgebaut. Der Katalysator enthielt 2,7 Gew.-% Re und 2.4 Gew.-% Platin.

### Vergleichsbeispiel 3

25 ml des Katalysators V2 wurden zu 0,1 - 1 mm großem Splitt gebrochen und in einen kontinuierlichen Rohrreaktor eingebaut. Der Feed bestand aus einer Maleinsäurelösung (10 % Maleinsäure) und wurde mit 25 g/h dosiert. 50 Nl/h Wasserstoff wurden zugegeben. Die Reaktionsbedingungen sind 160°C und 80 bar. Der Flüssigaustrag wurde mittels Gaschromatographie analysiert. Die Werte in Tabelle 4 sind jeweils als Ausbeuten in [%] pro mol Maleinsäure angegeben.

**Tabelle 4:**

| Bsp. | Umsatz MS [%] | BDO [%] | GBL [%] | THF [%] | BuOH [%] | BS/BSA [%] | C-Bilanz [%] |
|---|---|---|---|---|---|---|---|
| V3 | 100 | 19,7 | 0,0 | 20,3 | 0,1 | 0 | 41,3 |

### Vergleichsbeispiel 4: Katalysator V4

ZrO₂ Stränge (XZ 16509 der Firma Norton Process Prod. Corp., Akron (OH), USA, 82 m²/g, Porenvolumen 0,28 cm³/g) wurden zu 1.2 - 2 mm großem Splitt gebrochen. 200 g des Trägers wurden mit 39,24 g einer Platin-Nitrat-Lösung (16,25 Gew.-% Pt), die zuvor mit Wasser auf 160 ml aufgefüllt worden war, belegt. Nach 3 h Einwirkzeit wurde das Material bei 100°C und einem Druck von: 30-50 mbar 2 h getrocknet. Danach wurde 30 min unter 200 Nl/h N₂ auf 150°C aufgeheizt, 60 min gehalten und anschließend für 60 min bei dieser Temperatur ein Gemisch aus 100 Nl/h H₂ und 100 Nl/h N₂ zugeführt, binnen 30 min auf 300°C aufgeheizt und die Bedingungen für weitere 3 h gehalten. Sodann wurde der Vorkatalysator in dieser reduzierenden Atmosphäre auf 50°C und anschließend in reinem N₂ (200 Nl/h) bis auf Raumtemperatur abgekühlt. Das erhaltene Material wurde mit einem Gemisch von 10 Nl/h O₂ in 180 Nl/h N₂ während 14 h passiviert. Im nächsten Schritt wurde dieser Vorkatalysator mit 40 ml einer wässrigen Lösung von Re₂O₇ (10 Gew.-% Re₂O₇) unter Zusatz von weiteren 140 ml Wasser belegt. Die Einwirkzeit betrug 3 h. lm Anschluss wurde das Material bei 100°C und einem Druck von 30 - 50 mbar während 2 h getrocknet. Der fertige Katalysator wurde ebenso wie der Vorkatalysator reduziert. Der Katalysator enthielt 2,9 Gew.-% Re und 2,5 Gew.-% Pt.

### Vergleichsbeispiel 5

25 ml des Katalysators V4 wurden in einen kontinuierlichen Rohrreaktor eingebaut. Der Feed bestand aus einer Maleinsäurelösung (10 % Maleinsäure) und wurde mit 25 g/h dosiert. 50 Nl/h Wasserstoff wurden zugegeben. Es wurde bei 160°C und 80 bar hydriert. Der Flüssigaustrag wurde mittels Gaschromatographie analysiert. Die Werte in Tabelle 5 sind jeweils als Ausbeuten in [%] pro mol Maleinsäure angegeben.

**Tabelle 5:**

| Bsp. | Umsatz MS [%] | BDO [%] | GBL [%] | THF [%] | BuOH [%] | BS/BSA [%] | C-Bilanz [%] |
|---|---|---|---|---|---|---|---|
| V5 | 100 | 15,3 | 46,6 | 20,3 | 0,2 | 22,2 | 107,7 |

### Beispiel 7: Katalysator D

658 g ZrO₂ Stränge (XZ 16509 der Firma Norton Process Prod. Corp., Akron (OH), USA, 82 m²/g, Porenvolumen 0,28 cm³/g) wurden unter Rühren mit 27,3 g Re₂O₇ in 180 ml Wasser belegt. Das Material wurde bei 120°C getrocknet und danach in 30 min unter 200 Nl/h N₂ auf 150°C aufgeheizt und 60 min bei dieser Temperatur gehalten. Anschließend wurde für 60 min ein Gemisch aus 100 Nl/h H₂ und 100 Nl/h N₂ zugeführt, binnen 30 min auf 300°C aufgeheizt und diese Bedingungen für weitere 3 h gehalten. Sodann wurde der Vorkatalysator in dieser reduzierenden Atmosphäre auf 50°C und anschließend in reinem N₂ (200 Nl/h) bis auf Raumtemperatur abgekühlt. Abschließend wurde das Material mit einem Gemisch von 10 Nl/h Sauerstoff in 180 Nl/h N₂ während 14 h passiviert. Im nächsten Schritt wurden 131,3 g einer Platin-Nitrat-Lösung (16 Gew.-% Pt) mit 70 ml Wasser verdünnt und der Vorkatalysator.unter Rühren mit dieser Lösung getränkt. Nach Trocknung bei 120°C für 16 h wurde der fertige Katalysator ebenso wie der Vorkatalysator reduziert. Der Katalysator enthielt 2.8 Gew.-% Re und 2.9 Gew.-% Pt.

### Beispiel 8

25 ml des Katalysators D wurden zu 0,1 - 1 mm großem Splitt zerkleinert und in einen kontinuierlichen Rohrreaktor eingebaut. Der Feed bestand aus einer Maleinsäurelösung (10 Gew.-% Maleinsäure) und wurde mit 25 g/h dosiert. 50 Nl/h Wasserstoff wurden zugegeben. Es wurde bei 140°C und 80 bar hydriert. Der Flüssigaustrag wurde mittels Gaschromatographie analysiert. Die Werte in Tabelle 6 sind jeweils als Ausbeuten in [%] pro mol Maleinsäure angegeben.

**Tabelle 6:**

| Bsp. | Umsatz MS [%] | BDO [%] | GBL [%] | THF [%] | BuOH [%] | BS/BSA [%] | C-Bilanz [%] |
|---|---|---|---|---|---|---|---|
| 8 | 100 | 87,9 | 0,0 | 0,0 | 3,1 | 0,0 | 91,5 |

### Beispiel 9: Katalysator E

8,2 kg TiO₂-Pulver (Degussa P25 der Firma Degussa AG, Düsseldorf, 39 m²/g) wurden mit verdünnter Ameisensäure (47,1 g 85%ige HCOOH in 1350 g Wasser) versetzt und unter Zugabe von 2,86 kg Wasser 2 h. im Koller verknetet. Die fertige Masse wurde in einem Extruder mit 25 - 60 bar durch eine Matrize gepresst, so dass 4.5 mm starke Stränge entstanden. Die Stränge wurden bei 120°C 16 h getrocknet und dann zunächst 140 min bei 470°C und abschließend 3 h bei 500°C kalziniert. 8 g Re₂O₇ in 360 ml Wasser wurde im Rotationsverdampfer über 200 g des in 0.1 - 1 mm großen Splitt gebrochenen TiO₂-Trägers gegeben und nach 15 min Einwirkzeit der Katalysatorvorläufer bei 100°C und 10 mbar 2 h lang getrocknet. Das Material wurde am Drehrohrofen unter 50 Nl/h N₂ auf 300°C aufgeheizt und 1 h getempert. Anschließend wurden für 1,5 h zusätzlich 10 Nl/h Wasserstoff und für weitere zwei Stunden 25 Nl/h Wasserstoff dem Stickstoff hinzugefügt. Der Katalysatorvorläufer wurde unter N₂ abgekühlt und mit einem Gemisch aus 5 Nl/h Luft in 100 Nl/h N₂ bei Raumtemperatur über Nacht für die weitere Verarbeitung passiviert. Nachfolgend wurde ein Ansatz von 37,5 g einer Platin-Nitrat-Lösung (16 Gew.-% Pt) mit 320 ml Wasser verdünnt und über den Katalysatorvorläufer gegeben. Nach 15 min Einwirkzeit wurde bei 100°C und 10 mbar 2 h lang getrocknet. Das Material wurde am Drehrohrofen unter 50 Nl/h N₂ auf 300°C aufgeheizt und 1 h getempert. Anschließend wurden für 1,5 h zusätzlich 10 Nl/h Wasserstoff und für weitere zwei Stunden 25 Nl/h Wasserstoff dem Stickstoff hinzugefügt. Der Katalysator wurde unter N₂ abgekühlt und unter fließendem Stickstoff in Wasser ausgebaut. Der fertige Katalysator enthielt 2,4 Gew.% Platin und 2,9 Gew.-% Rhenium.

### Beispiel 10

12 ml des Katalysators E wurden mit 80 ml wässriger Maleinsäurelösung (5 Gew.-% Maleinsäure) in einen Autoklaven eingebaut. Es wurden bei Raumtemperatur 75 bar Wasserstoff aufgepresst. Die Temperatur wurde auf 160°C angehoben. Nach 1 h wurde die Reaktion abgebrochen. Der Flüssigaustrag wurde mittels Gaschromatographie analysiert. Die Werte in Tabelle 7 sind jeweils als Ausbeuten in [%] pro mol Maleinsäure angegeben.

**Tabelle 7:**

| Bsp. | Umsatz MS [%] | BDO [%] | GBL [%] | THF [%] | BuOH [%] | BS/BSA [%] | C-Bilanz [%] |
|---|---|---|---|---|---|---|---|
| 10 | 100 | 60,1 | 0,1 | 1,1 | 19,8 | 0,0 | 85,1 |

### Beispiel 11: Katalysator F

Zur Vorbehandlung werden 2000 ml des Aktivkohleträgers (Eco Sorb BG09, Splitt der Firma F.A.W. Jacobi AB, Stockholm, Schweden) 40 h mit 3800 g einer 41 %igen Phosphorsäure unter Rückfluss gekocht. Nach Abkühlen wird mit Wasser gewaschen, bis ein pH-Wert von 5 erreicht ist. Die Aktivkohle wird abschließend bei 120°C für 14 h getrocknet. 150 g des Trägers wurden mit 150 ml einer wässrigen Lösung von 15,2 g Re₂0₇, unter Rühren belegt und bei 120°C in 16 h getrocknet. Danach wurde das Material 30 min unter 200 Nl/h N₂ auf 150°C aufgeheizt und 60 min gehalten, anschließend wurde für 60 min ein Gemisch aus 100 Nl/h H₂ und 100 Nl/h N₂ zugeführt, binnen 30 min auf 300°C aufgeheizt und die Bedingungen für weitere 3 h gehalten. Sodann wurde der Vorkatalysator in dieser reduzierenden Atmosphäre auf 50°C und anschließen in reinem N₂ (200 Nl/h) bis auf Raumtemperatur abgekühlt. Abschließend wurde das Material mit einem Gemisch von 10 Nl/h Sauerstoff in 180 Nl/h N₂, während 14 h für die weitere Verarbeitung passiviert. Im nächsten Schritt wurden 31,3 g einer Platin-Nitrat-Lösung (16 Gew.-% Pt) mit 120 ml Wasser verdünnt und der Vorkatalysator mit dieser Lösung unter Rühren getränkt. Danach wurde das Material bei 120°C 16 h getrocknet. Der fertige Katalysator wurde ebenso wie der Vorkatalysator reduziert. Der Katalysator enthielt 6,8 Gew.-% Rhenium und 2,8 Gew.-% Platin.

### Vergleichsbeispiel 6: Katalysator V6

150 g des Trägers aus Beispiel 13 wurden mit 31,3 g einer Platin-Nitrat-Lösung (16 Gew.-% Pt), verdünnt mit 120 ml Wasser unter Rühren getränkt. Danach wurde das Material bei 120°C 16 h getrocknet und in 30 min unter 200 Nl/h N₂ auf 150°C aufgeheizt und 60 min gehalten. Anschließend wurde für 60 min ein Gemisch aus 100 Nl/h H₂ und 100 Nl/h N₂ zugeführt, binnen 30 min auf 300°C aufgeheizt-und die Bedingungen für weitere 3 h gehalten. Sodann wurde der Vorkatalysator in dieser reduzierenden Atmosphäre auf 50°C und anschließend in reinem N₂ (200 Nl/h) bis auf Raumtemperatur abgekühlt. Abschließend wurde das Material mit einem Gemisch von 10 Nl/h Sauerstoff in 180 Nl/h N₂ während 14 h passiviert. Im nächsten Schritt wurde das mit Platin vorbelegte Material mit 150 ml einer wässrigen Lösung von 15,2 g Re₂O₇ unter Rühren getränkt und bei 120°C in 16 h getrocknet. Der fertige Katalysator wurde ebenso wie der Vorkatalysator reduziert. Der Katalysator enthielt 6,5 Gew.-% Rhenium und 2,7 Gew.-% Platin.

### Beispiele 12 und 13, Vergleichsbeispiel 7

Jeweils 25 ml Katalysator wurden in einen kontinuierlichen Rohrreaktor eingebaut. Der Feed bestand aus einer wässrigen Maleinsäurelösung (10 Gew.-% Maleinsäure). 50 Nl/h Wasserstoff wurden zugegeben. Der Flüssigaustrag wurde mittels Gaschromatographie analysiert. Die Werte in Tabelle 8 sind jeweils als Ausbeuten in [%] pro mol Maleinsäure angegeben.

**Tabelle 8:**

| Bsp. | Kat | p [bar] | T [°C] | Feed [g/h] | Umsatz MS [%] | BDO [%] | GBL [%] | THF [%] | BuOH [%] | BS/BSA [%] | C-Bilanz [%] |
|---|---|---|---|---|---|---|---|---|---|---|---|
| 12 | F | 80 | 160 | 25 | 94,9 | 93,1 | 1,1 | 0,5 | 4,0 | 0,0 | 105,2 |
| 13 | F | 80 | 180 | 37,5 | 86,4 | 73,3 | 6,0 | 1,0 | 3,4 | 0,0 | 98,2 |
| V7 | V6 | 80 | 160 | 25 | 89,9 | 34,5 | 55,1 | 0,1 | 0,6 | 17,8 | 118,3 |

### Beispiel 14

25 ml des Katalysators A wurden in einen kontinuierlich zu betreibenden Rohrreaktor eingebaut. Der Feed bestand aus ein 10 gew.-%igen wässrigen Maleinsäurelösung und wurde mit 25 g/h zudosiert. Der Katalysator wurde mit dieser Lösung angefahren. Bei 160°C und 80 bar wurde mit 50 Nl/h Wasserstoff hydriert. Der flüssige Reaktionsaustrag wurde mittels Gaschromatographie nach verschiedenen Reaktionszeiten auf seinen 1,4-Butandiol-Gehalt hier analysiert und in Auftragung 1 gegen die Zeit aufgetragen.

### Beispiel 15

Die Hydrierung wurde analog Beispiel 16 durchgeführt, jedoch wurde Katalysator 12 h mit reinem Wasser angefahren. Sodann wurden Maleinsäurelösung und Wasserstoff zudosiert. Die ermittelten 1,4-Butandiolgehalte wurden als zweiter Graph in Auftragung 1 eingefügt.

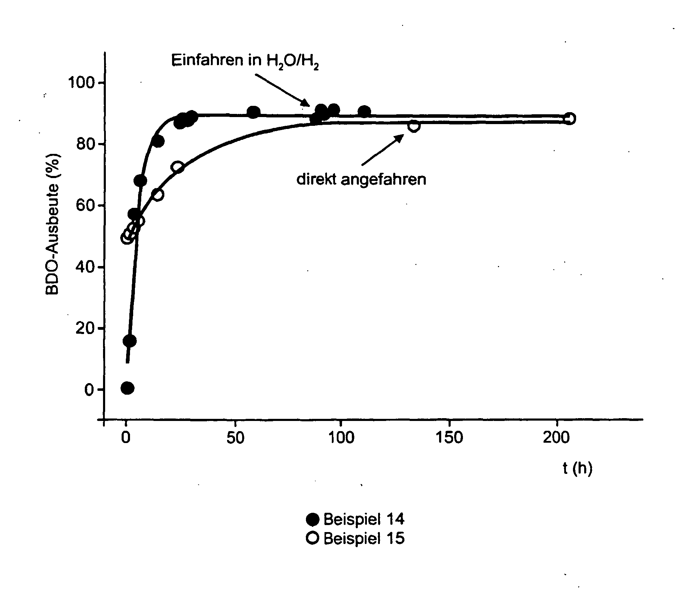

## Patentansprüche

1. Katalysator, enthaltend 0,1 bis 20 Gew.-% Rhenium und 0,05 bis 10 Gew.-% Platin, bezogen auf die Gesamtmasse des Katalysators, auf einem Träger, erhältlich durch ein Verfahren, bei dem man
a) den gegebenenfalls vorbehandelten Träger mit einer Lösung einer Rheniumverbindung behandelt,
b) trocknet und in einer reduktiven Atmosphäre bei 80 bis 600°C tempert,
c) mit einer Lösung einer Platinverbindung imprägniert und erneut trocknet.

2. Katalysator nach Anspruch 1, **dadurch gekennzeichnet, dass** der Träger ein Metalloxid, eine gegebenenfalls vorbehandelte Aktivkohle oder ein graphitischer Kohleträger, ein Nitrid, Silicid, Carbid oder Borid ist.

3. Katalysator nach Anspruch 2, **dadurch gekennzeichnet, dass** der Träger ausgewählt ist aus Titandioxid, Zirkondioxid, Hafniumdioxid, gegebenenfalls vorbehandelter Aktivkohle oder graphitischem Kohleträger.

4. Katalysator nach einem der Ansprüche 1 bis 3, **dadurch gekennzeichnet, dass** die reduktive Atmosphäre mindestens einen Anteil gasförmigen Ammoniak, Hydrazin, C₂- bis C₆-Olefin, Kohlenmonoxids und/oder Wasserstoff aufweist.

5. Katalysator nach einem der Ansprüche 1 bis 4, **dadurch gekennzeichnet, dass** nach Schritt b) der erhaltene Katalysatorrohling mit einem sauerstöffhaltigen Gas passiviert wird.

6. Katalysator nach einem der Ansprüche 1 bis 5, **dadurch gekennzeichnet, dass** er durch Anwendung einer reduzierend wirkenden Gasatmosphäre oder einer eines flüssigen Reduktionsmittels aktiviert wird.

7. Verfahren zur Herstellung von Alkoholen durch katalytische Hydrierung von Carbonylverbindungen zu Alkoholen, **dadurch gekennzeichnet, dass** ein Katalysator nach einem der Ansprüche 1 bis 6 verwendet wird.

8. Verfahren nach Anspruch 7, **dadurch gekennzeichnet, dass** die Carbonylverbindung ausgewählt ist aus Aldehyden, Carbonsäuren oder Estern, Anhydriden und/oder Lactonen.

9. Verfahren nach Anspruch 8, **dadurch gekennzeichnet, dass** die Carbonylverbindung ausgewählt ist aus Maleinsäure, Glutarsäure, Adipinsäure, Fumarsäure, Bernsteinsäure oder Estern oder Anhydriden davon, oder gamma-Butyrolacton, und zu 1,4-Butandiol hydriert wird.

10. Verfahren nach Anspruch 9, **dadurch gekennzeichnet, dass** die Carbonylverbindung ausgewählt ist aus Adipinsäure, 6-Hydroxycapronsäure oder Estern davon, oder Caprolacton, und zu 1,6-Hexandiol hydriert wird.

11. Verfahren nach einem der Ansprüche 7 bis 10, **dadurch gekennzeichnet, dass** die Hydrierung ist der Flüssigphase an fest angeordneten Katalysatoren bei einem Druck im Bereich von 20 bis 230 bar und einer Temperatur im Bereich von 80 bis 210□C durchgeführt wird.

12. Verfahren nach einem der Ansprüche 7 bis 11, **dadurch gekennzeichnet, dass** der mit dem Katalysator befüllte Hydrierreaktor mit Wasser oder einer verdünnten wässrigen Lösung der Carbonylverbindung unter Hydrierbedingungen angefahren wird.

## Claims

1. A catalyst comprising from 0.1 to 20% by weight of rhenium and from 0.05 to 10% by weight of platinum, based on the total mass of the catalyst, on a support, obtainable by a process in which
a) the optionally pretreated support is treated with a solution of a rhenium compound,
b) dried and heat-treated in a reductive atmosphere at from 80 to 600°C,
c) impregnated with a solution of a platinum compound and dried again.

2. The catalyst according to claim 1, wherein the support is a metal oxide, an optionally pretreated activated carbon or a graphitic carbon support, a nitride, silicide, carbide or boride.

3. The catalyst according to claim 2, wherein the support is selected from titanium dioxide, zirconium dioxide, hafnium dioxide, optionally pretreated activated carbon or a graphitic carbon support.

4. The catalyst according to any of claims 1 to 3, wherein the reductive atmosphere comprises at least a portion of gaseous ammonia, hydrazine, C₂- to C₆-olefin, carbon monoxide and/or hydrogen.

5. The catalyst according to any of claims 1 to 4, wherein, after step b), the catalyst blank obtained is passivated with an oxygenous gas.

6. The catalyst according to any of claims 1 to 5, which is activated by using a reducing gas atmosphere or a liquid reducing agent.

7. The process for preparing alcohols by catalytically hydrogenating carbonyl compounds to alcohols, which comprises using a catalyst according to any of claims 1 to 6.

8. The process according to claim 7, wherein the carbonyl compound is selected from aldehydes, carboxylic acids or esters, anhydrides and/or lactones.

9. The process according to claim 8, wherein the carbonyl compound is selected from maleic acid, glutaric acid, adipic acid, fumaric acid, succinic acid or esters or anhydrides thereof, or gamma-butyrolactone and is hydrogenated to 1,4-butanediol.

10. The process according to claim 9, wherein the carbonyl compound is selected from adipic acid, 6-hydroxycaproic acid or esters thereof, or caprolactone, and is hydrogenated to 1,6-hexanediol.

11. The process according to any of claims 7 to 10, wherein the hydrogenation is carried out in the liquid phase over fixed bed catalysts at a pressure in the range from 20 to 230 bar and a temperature in the range from 80 to 210°C.

12. The process according to any of claims 7 to 11, wherein the hydrogenation reactor charged with the catalyst is started up under hydrogenation conditions using water or a dilute aqueous solution of the carbonyl compound.

## Revendications

1. Catalyseur, contenant 0,1 à 20 % en poids de rhénium et 0,05 à 10 % en poids de platine, par rapport à la masse totale du catalyseur, sur un support, qui peut être obtenu par un procédé, selon lequel
a) le support éventuellement prétraité est traité avec une solution d'un composé de rhénium,
b) séché et recuit dans une atmosphère réductrice à 80 à 600 °C,
c) imprégné avec une solution d'un composé de platine et de nouveau séché.

2. Catalyseur selon la revendication 1, **caractérisé en ce que** le support est un oxyde métallique, un charbon actif éventuellement prétraité ou un support de charbon graphitique, un nitrure, un siliciure, un carbure ou un borure.

3. Catalyseur selon la revendication 2, **caractérisé en ce** le support est choisi parmi le dioxyde de titane, le dioxyde de zirconium, le dioxyde d'hafnium, le charbon actif éventuellement prétraité ou un support de charbon graphitique.

4. Catalyseur selon l'une quelconque des revendications 1 à 3, **caractérisé en ce que** l'atmosphère réductrice comporte au moins une fraction d'ammoniac gazeux, d'hydrazine, d'oléfine en C₂ à C₆, de monoxyde de carbone et/ou d'hydrogène.

5. Catalyseur selon l'une quelconque des revendications 1 à 4, **caractérisé en ce que** l'ébauche de catalyseur est passivée avec un gaz contenant de l'oxygène après l'étape b).

6. Catalyseur selon l'une quelconque des revendications 1 à 5, **caractérisé en ce qu'**il est activé par application d'une atmosphère gazeuse réductrice ou d'un réducteur liquide.

7. Procédé de fabrication d'alcools par hydrogénation catalytique de composés de carbonyle en alcools, **caractérisé en ce qu'**un catalyseur selon l'une quelconque des revendications 1 à 6 est utilisé.

8. Procédé selon la revendication 7, **caractérisé en ce que** le composé de carbonyle est choisi parmi les aldéhydes, les acides carboxyliques ou les esters, les anhydrides et/ou les lactones.

9. Procédé selon la revendication 8, **caractérisé en ce que** le composé de carbonyle est choisi parmi l'acide maléique, l'acide glutarique, l'acide adipique, l'acide fumarique, l'acide succinique ou leurs esters ou anhydrides, ou la gamme-butyrolactone, et hydrogéné en 1,4-butanediol.

10. Procédé selon la revendication 9, **caractérisé en ce que** le composé de carbonyle est choisi parmi l'acide adipique, l'acide 6-hydroxycapronique ou leurs esters, ou la caprolactone, et hydrogéné en 1,6-hexanediol.

11. Procédé selon l'une quelconque des revendications 7 à 10, **caractérisé en ce que** l'hydrogénation est réalisée en phase liquide sur des catalyseurs fixes à une pression dans la plage allant de 20 à 230 bar et à une température dans la plage allant de 80 à 210 °C.

12. Procédé selon l'une quelconque des revendications 7 à 11, **caractérisé en ce que** le réacteur d'hydrogénation rempli avec le catalyseur est démarré avec de l'eau ou une solution aqueuse diluée du composé de carbonyle en conditions d'hydrogénation.
